# EUROPEAN PATENT SPECIFICATION

(11) **EP 3 370 689 B1**
(45) Date of publication and mention of the grant of the patent: **04.10.2023**
(21) Application number: 16798815.3
(22) Date of filing: 31.10.2016
(51) Int. Cl.: A61K 8/49, A61Q 17/00, A61K 8/81, A61Q 19/08, A61K 8/14

(54) **COMPOSITION FOR PROTECTING THE SKIN AGAINST THE EFFECTS OF POLLUTION AND USE THEREOF**
ZUSAMMENSETZUNG ZUM SCHUTZ DER HAUT GEGEN DIE AUSWIRKUNGEN VON VERSCHMUTZUNG UND VERWENDUNG DAVON
COMPOSITION POUR PROTÉGER LA PEAU CONTRE LES EFFETS DE LA POLLUTION ET LEUR UTILISATION

(30) Priority: 02.11.2015 EP 15382538
(43) Date of publication of application: 12.09.2018
(73) Proprietor: Lubrizol Advanced Materials, Inc., Cleveland, OH 44141-3247 (US)
(72) Inventor: CAÑADAS, Elena, 08850 Gavà Barcelona (ES); OLLAGNIER, Marie, 92977 Paris La Defense (FR)
(74) Representative: Bradley, Josephine Mary
(86) International application number: PCT/IB2016/056549
(87) International publication number: WO 2017/077442

(56) References cited:
- EP-A1- 1 002 533
- EP-A1- 1 046 390
- EP-A1- 1 336 403
- EP-A2- 2 462 919
- WO-A1-02/083089
- WO-A1-2011/101153
- WO-A2-2004/060273
- US-A1- 2003 165 444
- US-A1- 2010 063 139
- DATABASE GNPD [Online] MINTEL; October 2015 (2015-10), "Wrinkle Correcting Day Cream SPF 15", XP002754845, Database accession no. 3501599

## Description

### FIELD OF THE INVENTION

The disclosed technology relates to the use of a composition for protecting the skin against adverse effects of pollution.

### BACKGROUND OF THE INVENTION

The major function of the skin is to form a barrier between the inside of an organism and the environment. The skin acts to protect the organism from chemicals, ultraviolet light, mechanical insults and pathogenic micro-organisms, for example. Also, the skin acts to provide an efficient permeability barrier that prevents the loss of water and electrolytes [1].

Air and water pollutants can have detrimental effects on the skin. Major air pollutants which affect the skin include polycyclic aromatic hydrocarbons, volatile organic compounds, nitrogen oxides (NOx), particulate matter and cigarette smoke. The actions of various air pollutants may be amplified in the presence of other air pollutants and with the interaction of UV radiation, and may form major active comp-nents of pro-oxidant smog [2]. Alterations that disturb the skin barrier function, in either stratum corneum lipid metabolism or protein components of the corneocytes, are involved in the development of various skin diseases. Air pollutants may induce severe interference of normal function of lipids, DNA and/or proteins of the human skin via oxidative damage leading to skin aging, inflammatory or allergic conditions such as atopic dermatitis, psoriasis and acne, and skin cancer [2]. Indeed, there is growing evidence that environmental factors like air pollution may contribute to skin aging [3]. Skin aging results from the combined action of intrinsic and extrinsic factors. From a preventative point of view, the latter are of particular interest because they can be modified more easily. In the case of skin, extrinsic skin aging can be clearly distinguished from intrinsic skin aging at a clinical, histological and molecular level. Clinical symptoms of extrinsic skin aging include coarse wrinkles, irregular pigment spots, and elastosis.

Particulate matter comprises complex mixtures containing metals, minerals, organic toxins, and/or biological materials. Particulate matter has been shown to induce skin aging, allergic reactions, and delay the wound healing of the skin by topical exposure. The components of particulate matter that receive the most attention for causing health problems are heavy metals and polycyclic aromatic hydrocarbons [4].

The stratum corneum and tight junctions (a type of intercellular junction) in the epidermis function as a permeation barrier against the penetration of molecules. Permeation barrier deterioriation by particulate matter may accelerate drug absorption into the skin, augmenting the risk of over absorption. Studies have found that proteins related to the barrier function (cytokeratin, filaggrin and E-cadherin) can be diminished after pollutant treatment [4]. Cytokeratin serves as part of the cytoskeleton networks and membrane-associated lipid rafts, which are in response to xenobiotic stress. A down regulation of cytokeratin by particulate matter results in fragility of the skin's epithelium. The loss of cytokeratin also leads to the cytolysis and apoptosis and disturbance of the epidermal barrier. Filaggrin is a structural protein, which is postulated to participate in the mechanical strength and integrity of the stratum corneum and epidermal barrier function. Filaggrin deficiency results in the loss of natural moisture factor in the stratum corneum. Studies have found the expression of E-cadherin, a tight junction-related protein and filiggrin to be impaired following pollutant treatment. Loss of tight junctions not only diminishes the barrier property, but also creates wrinkling [4].

In addition to the damage caused by the intact particles of the particulate matter, skin damage might be caused by the chemicals released from the particles. Particulate matter can undergo dissolution when it comes into contact with human tissue. This dissolution may lead to release of components from the particles into the skin. The hydrocarbon contaminants can bind to aryl hydrocarbon receptors, which are largely present in the innate cells of interface organs such as the gut and skin. Polycyclic aromatic hydrocarbons can disrupt the plasma membrane, changing cell physiology and evoking the death of cells, and inhibit skin re-epithelialization [4]. Organic chemicals and metals are capable of localizing in mitochondria and producing reactive oxygen species. In particular, polycyclic aromatic hydrocarbons can be adsorbed on the surface of particulate matter suspended in air in urban area and can activate xenobiotic metabolism, which converts the polycyclic aromatic hydrocarbons to quinones. Quinones are redox-cycling chemicals, which produce reactive oxygen species and are therefore thought to be key compounds in particulate matter toxicity.

Reactive oxygen species production can overwhelm the skin' defenses by quickly depleting the enzymatic (glutathione peroxidase, glutathione reductae, superoxide dismutase, catalase) and non-enyzmatic (vitamin E, vitamin C and glutathione) anitoxidant capacity, thus leading to deleterious effects. Reactive oxygen species also stimulate the release of pro-inflammator mediators from a variety of skin cells. Oxidative stress initiates complex biological processes in various layers of the skin, which can result in transient or permanent genetic damage [5]. The skin is continuously and simultaneously exposed to several oxidative stressors that can have additive, if not synergistic, effects. In one of the few studies that investigated a possible additive/synergistic effect of pollutant/stressors on skin, UV and O₃ were found to have an additive effect on antioxidant depletion (vitamin E) on lipid peroxidation levels, which could, in turn, lead to addition additive effects of these stressors. [5]

There is a need for a product that can alleviate or prevent adverse effects of pollution (air and water) on the skin, hair, mucous membranes and/or nails. In particular, there is a need for a product that is effective against more than one of the adverse effects of pollution, especially air pollution, on the skin, hair, mucous membranes and/or nails.

EP1046390A1 (to Calgon Corporation, filed on 19 April 2000) discloses the use of an anionic dispersant in an aqueous composition at a pH of between 2 and 6 for removing metals, metal ions, metal salts, environmental dirt and sebum from hair. The metal build up in hair is described as originating from well water, surface water, water from pipes where the water supplies are chlorinated periodically and from certain algicides add frequently to swimming pools. The anionic dispersant may be a polymer made from at least one monomer selected from the group consisting of the following; acrylic acid, methacrylic acid, 2-acrylamido-2-propane sulfonic acid (AMPSA), 2-methacrylamido-2-propane sulfonic acid, itaconic acid, crotaonic acid, fumaric acid, maleic anhydride acid, isocrotonic acid, aconitic acid (cis or trans), mesaconic acid, sinapinic acid, undecylenic acid angelic acid, canellic acid, or hydroxyacrylic acid, sulfonated styrene, vinyl sulfonate, 3-allyloxy-2-hydroxy propyl sulfonic acid, vinyl phosphonic acid, and sulphenoxy methallyl ether, existing either as free acids or partially neutralized salts, acrolein, acrylamide, acrylonitrile, the esters of acrylic and methacrylic acids, dimethlaminoethyl methacrylate, vinylpyrrolidone, vinylcaprolactam, ethylene, propylene, isobutylene, diisbutylene, vinyl acetate, styrene, α-methyl styrene, methyl vinyl ketone, and hydroxy propyl acrylate. A preferred anionic dispersant is a coplymer comprised of about 60 wt % acrylic acid and 40 wt % 2-methacrylamido-2-propane sulfonic acid available from Calgon Corporation as pHREEGuard 4500. This document is not concerned with the effects of pollution on the skin.

EP1002533B1 (to Lipotec, S.A., filed on 10 September 1999) discloses the use of a compound of general formula (A) or (B): 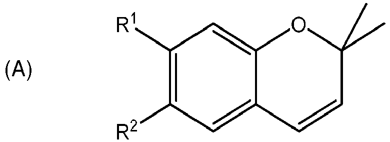 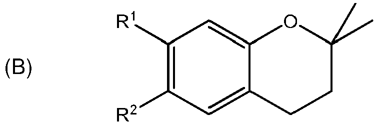 wherein R¹ and R² are identical or different and are independently selected from the group consisting of -OH, -CH₃, CF₃CH₂O- and CH₃O-, in the manufacture of a pharmaceutical composition for the oral or topical treatment of diseases associated with oxidative reactions or reactions induced by free radicals.

Dimethylmethoxy chromanol (also known as 7-methoxy-2,2-dimethylchroman-6-ol; CAS no. 83923-51-7, Lipochroman-6) is known as an inhibitor of peroxynitrile-induced lipid peroxidation and as an effective reactive nitrogen species and reactive oxygen species scavenger, therefore giving protection against free radicals. It is known for use in the prevention of skin damage induced by reactive nitrogen species and in helping avoid premature skin aging.

There is a need to provide an alternative to Lipochroman-6 or a product which outperforms same in protecting the skin against premature aging.

The present invention sets out to solve some or all of the above-identified problems and meet some or all of the above-identified needs.
[1] Feingold K., Elias P., The important role of lipids in the epidermis and their role in the formation and maintenance of the cutaneous barrier. Biochim Biophys Acta. 2014 Mar;1841(3):279.
[2] Eleni Drakaki, Clio Dessinioti and Christins V Antoniou, Air Pollution and Skin, Frontiers in Environmental Science, May 2014, Volume 2, Article 11.
[3] Vierkötter A., Schikowski T., Ranft U., Sugiri D., Matsui M., Krämar U., Krutmann J., Airborne particle exposure and extrinsic skin aging.; J. Invest. Dermatol. 2010, 130(12):2719-26.
[4] Pan T., Wang P., Aljuffali I., Huang C., Lee C., Fang J.. The impact of uraban particulate pollution on skin barrier function and the subsequent drug absorption. J. Dermatol. Sci. 2015 Apr;78(2015):51-60.
[5] Valacchi G., Sticozzi C., Pecorelli A., Cervellati C., Maioli E., Cutaneous response to environmental stressors. Ann. N. Y. Acad. Sci. 2012: 1271: 75-81.

### SUMMARY OF THE INVENTION

The invention is defined in the appended claims. In embodiments, the invention employs a composition comprising a compound (I) of formula (A) or formula (B): 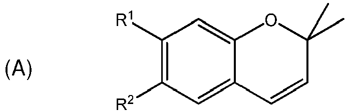 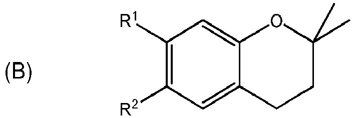 wherein R¹ and R² are identical or different and are independently selected from the group consisting of -OH, -CH₃, CF₃CH₂O- and CH₃O-; and an anionic polymer (II) which is a copolymer of acrylic acid or methacrylic acid and 2-acrylamido-2-methylpropane sulfonic acid, as defined in the appended claims.

It has been found that the combination of compound (I) and anionic polymer (II) of the invention is particularly effective in protecting the skin against more than one of the adverse effects of pollution. It is believed that the relationship between compound (I) and anionic polymer (II) can be synergistic in the sense that, when combined, an anti-pollution effect is obtained which is greater than the sum of the anti-pollution effects compound (I) and polymer (II) can produce individually. Alternatively, the relationship between compound (I) and anionic polymer (II) can be such that, when combined, an enhanced anti-pollution effect is obtained that is greater than the anti-pollution effect either compound (I) or polymer (II) can produce individually. By anti-pollution effect is meant a protective effect whereby an adverse effect of pollution on the skin is alleviated or prevented.

Adverse effects of pollution on the skin can manifest themselves in the form of premature aging of the skin, damage to the skin due to the presence of oxidants and free radicals, dullness of the skin, lack of a healthy look, lack of radiance, dryness and roughness, the presence of pigment spots, the presence of wrinkles, a lack of clean-iliness (build up of impurities/city grime), increased sensitivity of skin, skin inflammatory reactions, skin acne, alteration of activity of the skin's epidermal and dermal structure; decrease in skin oxygen levels, decrease in the skin's ability to breath, effects on the density of the skin's collagen network.

The invention relates to the use of the combination of compound (I) and anionic polymer (II) for the cosmetic treatment and/or care of the skin, where the treatment and/or care includes the treatment and/or care of the adverse effects of pollution on the skin, as defined in the appended claims.

### DESCRIPTION OF THE INVENTION

The preferred embodiments as set out below are applicable to all the above-mentioned aspects of the invention.

### Definitions

In the context of this invention "skin" is understood to be the layers which comprise it, from the uppermost layer or stratum corneum to the lowermost layer or hypodermis, both inclusive. These layers are composed of different types of cells such as keratinocytes, fibroblasts, melanocytes and/or adipocytes among others. In the context of this invention, the term "skin" includes the scalp. The term "skin" includes human skin.

The term "treatment", as used in the context of this specification when it is not accompanied by the qualifications "cosmetic" or "non-therapeutic", means the administration of a compound according to the invention to alleviate or cure a disease or disorder, or reduce or eliminate one or more symptoms associated with this disease or disorder, or alleviate or eliminate the physiological consequences of the disease or disorder.

Where the term "treatment" is accompanied by the qualification "cosmetic", it means that the treatment is non-therapeutic and has the aim of improving the aesthetic appearance of the skin and includes improving the properties of the skin such as, but not restricted to, the level of hydration, elasticity, firmness, shine, tone or texture, which properties affect the cosmetic appearance of the skin. Where the term "care" is accompanied by the qualification "cosmetic", it means that the care is non-therapeutic and has the aim of maintaining the aesthetic appearance of the skin. The term "care" in the context of this specification refers to the maintenance of the properties of the skin. The properties of the skin are subject to improvement and maintenance through cosmetic treatment and/or carebethin healthy subjects.

The term "prevention", as used for this invention, refers to the ability of the active of the invention to prevent, delay or hinder the appearance or development of a symptom or manifestation of an adverse effect of pollution on skin.

In the context of this invention, the term "aging" refers to the changes experienced by the skin with age (chronoaging) or through exposure to the sun (photoaging) or to environmental agents such as tobacco smoke, extreme climatic conditions of cold, heat, or wind, chemical contaminants or pollutants, and includes all the external visible and/or perceptible changes through touch, such as and not restricted to, the development of discontinuities on the skin such as wrinkles, fine lines, furrows, irregularities or roughness, increase in the size of pores, loss of elasticity, loss of firmness, loss of smoothness, loss of the capacity to recover from deformation, sagging of the skin such as sagging cheeks, the appearance of bags under the eyes or the appearance of a double chin, among others, changes to the color of the skin such as marks, reddening, bags under the eyes or the appearance of hyperpigmented areas such as age spots or freckles among others, anomalous differentiation, hyperkeratinization, elastosis, keratosis, hair loss, orange-peel skin, loss of collagen structure and other histological changes of the stratum corneum, of the dermis, epidermis, vascular system (for example the appearance of spider veins or telangiectasias) or of those tissues close to the skin, among others. The term "photoaging" groups together the set of processes due to the prolonged exposure of the skin to ultraviolet radiation which result in the premature aging of the skin, and present the same physical characteristics as aging, such as and not restricted to, flaccidity, sagging, changes to the color or irregularities in the pigmentation, abnormal and/or excessive keratinization. The changes of the skin due to aging (e.g. chronoaging, photoaging and/or environmental aging) are also referred to herein as the symptoms or signs of skin aging.

Disclosed herein is a composition comprising a compound (I) of formula (A) or formula (B): 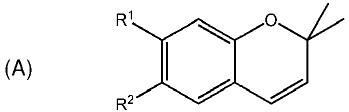 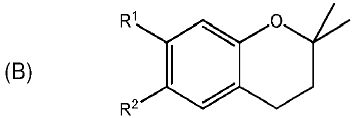 wherein R¹ and R² are identical or different and are independently selected from the group consisting of -OH, -CH₃, CF₃CH₂O- and CH₃O-; and an anionic polymer (II) which is a copolymer of acrylic acid or methacrylic acid and 2-acrylamido-2-methylpropane sulfonic acid.

Surprisingly, the inventors have found that the combination of compound (I) and anionic polymer (II) is active as protective agent for the skin against adverse effects of pollution. In particular, the combination is active in protecting the skin against a multiplicity of the adverse effects that pollution can have on the skin. Further, in particular, the combination is active in protecting the skin against a multiplicity of the adverse effects that air pollution can have on the skin.

Component (I) is a 2,2-dimethyl chromene of formula (A) or a 2,2-dimethyl chomane of formula (B) as defined above. These compounds and their preparation are described in EP1002533B1 (to Lipotec, S.A., filed on 10 September 1999). In one embodiment, compound (I) is a compound of formula (B) wherein R¹ and R² are identical or different and are independently selected from the group consisting of -OH, -CH₃, CF₃CH₂O- and CH₃O. In one embodiment, component (I) is a compound of formula (B), wherein R¹ is CH₃O- and R² is -OH.

Anionic polymer (II) is a copolymer of acrylic acid or methacrylic acid and 2-acrylamido-2-methylpropane sulfonic acid.

The anionic polymer is water soluble and can be obtained using conventional radical polymerisation procedures such as those described in EP1046390A1.

In one embodiment, component (II) is a copolymer of acrylic acid and 2-acryloamido-2-methylpropane sulfonic acid.

The anionic polymer (II) has a low molecular weight which can be less than 1, 000, 000 (Mw).

In one embodiment, the composition comprises for compound (I), a compound of formula (B) wherein R¹ and R² are identical or different and are independently selected from the group consisting of -OH, -CH₃, CF₃CH₂O- and CH₃O and anionic polymer (II), a copolymer of: acrylic acid or methacrylic acid; and 2-acryloamido-2-methylpropane sulfonic acid.

In one embodiment, the composition comprises for compound (I), a compound of formula (B) wherein R¹ is CH₃O- and R² is -OH and, for anionic polymer (II), a copolymer of acrylic acid and 2-acryloamido-2-methylpropane sulfonic acid.

The composition can contain at least one cosmetically or pharmaceutically acceptable excipient, adjuvant and/or ingredient.

The composition can be incorporated into cosmetic or pharmaceutical delivery systems and/or sustained release systems.

The term "delivery systems" relates to a diluent, adjuvant, vehicle or additives with which the composition of the invention is administered. The simplest existing delivery system consists of solvent or carrier in liquid form, such as water, oils or surfactants, including those of petroleum, animal, plant or synthetic origin, such as and not restricted to, peanut oil, soybean oil, mineral oil, sesame oil, castor oil, polysorbates, sorbitan esters, ether sulfates, sulfates, betaines, glycosides, maltosides, fatty alcohols, nonoxynols, poloxamers, polyoxyethylenes, polyethylene glycols, dextrose, glycerol, digitonin and similar. A person skilled in the art is aware of the diluents, adjuvants or additives that can be used as such and as components of more complex delivery systems in which the composition of the invention can be administered.

The term "sustained release" is used in a conventional sense to describe a complex delivery system which provides the gradual release of a compound over a period of time. In one embodiment, the delivery system provides relatively constant compound release levels over a period of time.

Examples of delivery systems able to perform controlled or sustained release include liposomes, mixed liposomes, oleosomes, niosomes, ethosomes, milliparticles, microparticles, nanoparticles and solid lipid nanoparticles, nanostructured lipid carriers, sponges, cyclodextrins, vesicles, micelles, mixed micelles of surfactants, surfactant-phospholipid mixed micelles, millispheres, microspheres and nanospheres, lipospheres, millicapsules, microcapsules and nanocapsules, as well as microemulsions and nanoemulsions, which can be added to achieve a greater penetration of the active ingredient of the invention. In one embodiment, the delivery or sustained release systems are chosen from liposomes, surfactant-phospholipid mixed micelles and microemulsions, water-in-oil microemulsions with an internal reverse micelle structure, and microemulsions containing nanocapsules.

The sustained release system can be prepared by methods known in the prior art, and the compositions which contain them can be administered, for example, by topical or transdermal administration, including adhesive patches, non-adhesive patches, occlusive patches and microelectric patches. In one embodiment, the sustained release system should release a relatively constant quantity of the combination of active components (compound (I) and anionic polymer (II)) of the invention. The amount of the combination of active components contained in the sustained release system will depend, for example, on where the composition is to be administered, the kinetics and duration of the release of the composition, as well as the nature of the condition, disorder and/or disease to be treated or prevented.

In some embodiments, compound (I) and anionic polymer (II) are present in the composition in quantities effective to provide protection of the skin against the adverse effects of pollution. Compound (I) can be present in an amount of from 0.01 to 0.10 wt %, 0.03 to 0.08 wt %, or 0.04 to 0.06 wt %, based on the total weight of the composition. Anionic polymer (II) can be present in an amount of from 0.05 to 0.5 wt%, 0.15 to 0.4 wt %, or 0.2 to 0.3 wt%, based on the total weight of the composition. In one embodiment, compound (I) and anionic polymer (II) are present in quantities effective to provide a synergetic effect in protecting the skin against the adverse effects of pollution. The effective quantities of compound (I) and anionic polymer (II) in the compostion, as well as its dosage, will depend on numerous factors, which can include age, condition of the subject, the nature or severity of the adverse effect of pollution on the skin to be treated and/or cared for, the route and frequency of administration of the composition. In one embodiment, compound (I) and anionic polymer (II) are present in the composition in a ratio of 1:5 by weight.

In some embodiments, the compositions are concentrates. In these embodiments, typically, the compositions comprise the active components of the invention (compound (I) and anionic polymer (II)) and a carrier or diluent such as a liposomal fluid. In these embodiments, compound (I) can be present in an amount of from 0.1 to 10 wt %, 0.5 to 5 wt %, or 0.8 to 1.5 wt %, and anionic polymer (II) can be present in an amount of from 0.5 to 10 wt%, 2.5 to 8 wt %, or 3 to 6 wt%, based on the total weight of the composition. In one embodiment, compound (I) and anionic polymer (II) are present in the concentrate in a ratio of 1:5 by weight.

The composition can also be adsorbed on solid organic polymers or solid mineral supports, such as and not restricted to, talc, bentonite, silica, starch or maltodextrin among others.

The compositions can also be incorporated into fabrics, non-woven fabrics or medical devices which are in direct contact with the skin, thus releasing the combination of actives of the invention whether by biodegradation of the binding system to the fabric, non-woven fabric or medical device, or due to the friction between them and the body, due to body moisture, the skin's pH or body temperature. Furthermore, the composition of the invention can be incorporated into the fabrics and non-woven fabrics used in the manufacture of garments that are in direct contact with the body.

Examples of fabrics, non-woven fabrics, garments, medical devices and means for immobilizing the compounds to them, among which are the delivery systems and/or the sustained release systems described above, can be found in the literature and are known in the prior art *[*Schaab C.K. (1986) HAPPI May 1986*;* Nelson G., "Application of microencapsulation in textiles", (2002), Int. J. Pharm., 242(1-2), 55-62*; "*Biofunctional Textiles and the Skin" (2006) Curr. Probl. Dermatol. v.33, Hipler U.C. and Elsner P., eds. S. Karger AG, Basel, Switzerl*and;* Malcolm R.K. et al., "Controlled release of a model antibacterial drug from a novel self-lubricating silicone biomaterial", (2004), J. Cont. Release, 97(2), 313-320*].* The preferred fabrics, non-woven fabrics, garments and medical devices are bandages, gauzes, t-shirts, socks, tights, underwear, girdles, gloves, diapers, sanitary napkins, dressings, bedspreads, wipes, adhesive patches, non-adhesive patches, occlusive patches, microelectric patches and/or face masks.

The compositions can be used in different types of compositions for topical or transdermal application, optionally including acceptable excipients necessary for formulating the desired administration form.

The compositions can be for topical or transdermal application and can be produced in any solid, liquid or semi-solid formulation, such as and not restricted to, creams, multiple emulsions such as and not restricted to, oil and/or silicone in water emulsions, water-in-oil and/or silicone emulsions, water/oil/water or water/silicone/water type emulsions, and oil/water/oil or silicone/water/silicone type emulsions, anhydrous compositions, aqueous dispersions, oils, milks, balsams, foams, lotions, gels, cream gels, hydroalcoholic solutions, hydroglycolic solutions, hydrogels, liniments, sera, soaps, shampoos, conditioners, serums, polysaccharide films, ointments, mousses, pomades, powders, bars, pencils and sprays or aerosols (sprays), including leave-on and rinse-off formulations. These topical or transdermal application formulations can be incorporated, using techniques known by the person skilled in the art, into different types of solid accessories such as and not restricted to, bandages, gauzes, t-shirts, socks, tights, underwear, girdles, gloves, diapers, sanitary napkins, dressings, bedspreads, wipes, adhesive patches, non-adhesive patches, occlusive patches, microelectric patches or face masks, or they can be incorporated into different make-up products such as make-up foundation, such as fluid foundations and compact foundations, make-up removal lotions, make-up removal milks, under-eye concealers, eye shadows, lipsticks, lip protectors, lip gloss and powders, among others.

The compositions may include agents which increase the percutaneous absorption of the compounds of this invention, for example and not restricted to, dimethyl sulfoxide, dimethylacetamide, dimethylformamide, surfactants, azone (1-dodecylazacycloheptane-2-one), alcohol, urea, ethoxydiglycol, acetone, propylene glycol or polyethylene glycol, among others. Furthermore, the compositions of this invention can be applied to local areas to be treated by means of iontophoresis, sonophoresis, electroporation, microelectric patches, mechanical pressure, osmotic pressure gradient, occlusive cure, microinjections or needle-free injections by means of pressure, such as injections by oxygen pressure, or any combination thereof, to achieve a greater penetration of the composition. The application area will be determined by the nature of the condition, disorder and/or disease to be treated and/or prevented.

Among the acceptable excipients, adjuvants and/or ingredients contained in the compositions described in this invention are additional ingredients commonly used in compositions such as and not restricted to, agents which diminish the sebum production, anti-seborrheic agents, mattifying agents, anti-acne agents, agents stimulating the synthesis of dermal or epidermal macromolecules and/or capable of inhibiting or preventing their degradation, other collagen synthesis-stimulating agents, other elastin synthesis-stimulation agents, decorin synthesis-stimulation agents, laminin synthesis-stimulation agents, defensin synthesis-stimulating agents, chaperone synthesis-stimulating agents, cAMP synthesis-stimulating agents, agents that modulate AQP-3, agents that modulate aquaporin synthesis, proteins from the aquaporin family, hyaluronic acid synthesis-stimulating agents, glycosaminoglycan synthesis-stimulating agents, fibronectin synthesis-stimulating agents, sirtuin synthesis-stimulating agents, heat shock proteins, heat shock protein synthesis-stimulating agents, agents which inhibit neuronal exocytosis, other anticholinergic agents, agents which inhibit muscular contraction, other anti-aging agents, other anti-wrinkle agents, antiperspirant agents, anti-inflammatory agents and/or analgesics, anti-itching agents, calming agents, anesthetic agents, inhibitors of acetylcholine-receptor aggregation, agents that inhibit acetylcholinesterase, skin relaxant agents, other melanin synthesis inhibiting agents, whitening or depigmenting agents, NO-synthase inhibiting agents, 5α-reductase inhibiting agents, lysyl- and/or prolyl hydroxylase inhibiting agents, antioxidants, free radical scavengers and/or agents against atmospheric pollution, reactive carbonyl species scavengers, anti-glycation agents, antihistamine agents, antiviral agents, antiparasitic agents, emulsifiers, emollients, organic solvents, liquid propellants, skin conditioners, humectants, substances that retain moisture, alpha hydroxy acids, beta hydroxy acids, moisturizers, epidermal hydrolytic enzymes, vitamins, amino acids, proteins, pigments or colorants, dyes, biopolymers, gelling polymers, thickeners, surfactants, softening agents, emulsifiers, binding agents, preservatives, agents able to reduce or treat the bags under the eyes, exfoliating agents, keratolytic agents, desquamating agents, antimicrobial agents, antifungal agents, fungistatic agents, bactericidal agents, bacteriostatic agents, agents stimulating the synthesis of lipids and components of the stratum corneum, ceramides, fatty acids, other agents that inhibit collagen degradation, agents that inhibit matrix metalloproteinases,other agents that inhibit elastin degradation, agents that inhibit serine proteases such as kallikreins, leukocyte elastase or cathepsin G, agents stimulating fibroblast proliferation, agents stimulating keratinocyte proliferation, agents stimulating adipocyte proliferation, agents stimulating melanocyte proliferation, agents stimulating keratinocyte differentiation, agents stimulating or delaying adipocyte differentiation, antihyperkeratosis agents, comedolytic agents, anti-psoriasis agents, DNA repair agents, DNA protecting agents, stem cell protecting agents, stabilizers, agents for the treatment and/or care of sensitive skin, firming agents, anti-stretch mark agents, binding agents, lipolytic agents or agents stimulating lipolysis, adipogenic agents, agents modulating PGC-1α expression, agents modulating the activity of PPARγ, agents which increase or reduce the triglyceride content of adipocytes, anti-cellulite agents, agents which inhibit the activity of PAR-2, agents stimulating healing, coadjuvant healing agents, agents stimulating reepithelialization, coadjuvant reepithelialization agents, cytokine growth factors, agents acting on capillary circulation and/or microcirculation, agents stimulating angiogenesis, agents stimulating communication between neighboring cells, agents which increase connexin expression, agents that inhibit vascular permeability, venotonic agents, agents acting on cell metabolism, agents which improve dermal-epidermal junction, agents inducing hair growth, hair growth inhibiting or retardant agents, hair loss retardant agents, preservatives, perfumes, odor absorbents and/or body odor masking deodorants, chelating agents, plant extracts, essential oils, marine extracts, agents obtained from a biotechnological process, mineral salts, cell extracts, sunscreens and organic or mineral photoprotective agents active against ultraviolet A and/or B rays and/or infrared A rays, or mixtures thereof, provided that they are physically and chemically compatible with the rest of components in the composition and particularly with the product. Likewise, the nature of these additional ingredients should not unacceptably alter the benefits of the composition of this invention. The nature of these additional ingredients can be synthetic or natural, such as plant extracts, or come from a biotechnological process, or from a combination of a synthetic process and a biotechnological process. Additional examples can be found in CTFA International Cosmetic Ingredient Dictionary & Handbook, 12th Edition (2008*).* In the context of this invention, biotechnological process is understood to be any process to produce the active ingredient, or part of it, in an organism, or in part of it.

In one embodiment, the composition contains:
- between 0.01% (in weight) and 20% (in weight) of the compound (I);
- between 0.01% (in weight) and 20% (in weight) of the anionic polymer (II)
- between 0.1% (in weight) and 20% (in weight) of an humectant or moisturizing agent selected from the group of (INCI Names) Glycerin, Propanediol, Propylene Glycol, Butylene Glycol, Pentylene Glycol, Caprylyl Glycol, Lactic Acid, Urea, Sodium Hyaluronate, Methyl Gluceth-20.
- between 0.1% (in weight) and 20% (in weight) of an emollient for solubilizing compound (I) selected from the group of (INCI Names) diisopropyladipate, diiso-propylsebacate, mineral oil (paraffinum liquidum), caprylic/capric triglyceride, C12-C15 alkyl benzoate, isohexadecane, ethyelhexyl methoxycinnamate.
- between 0.1% (in weight) and 20% (in weight) of an emollient or skin conditioning agent selected from the group of (INCI Names) Dimethicone, Squalane, Lanolin, Tocopheryl Acetate, Panthenol, Butyrospermum Parkii Butter, Retinyl Palmitate, Retinol.
- between 0.1% (in weight) and 20% (in weight) of an emulsifier selected from the group of (INCI Names) Glyceryl Stearate, Cetearyl Alcohol, Lecithin, Potassium Cetyl Phosphate, Methyl Glucose Sesquistearate, Stearic Acid, PEG-20 methyl Glucose Sesquistearate, Behenyl Alcohol .
- between 0.1% (in weight) and 20% (in weight) of a stabilizer selected from the group of (INCI Names) Xanthan Gum, Sclerotium Gum, Carbomer, Acylates/C10-30 Alkyl Acrylate Crosspolymer,Cellulose Gum, Hydroxyethylcelullose.

The components of the composition add up to 100 % by weight. The composition of the invention can have a pH of between 3.5 and 11.

The invention provides for the use of the combination of compound (I) and anionic polymer (II) for the cosmetic treatment and/or care of the skin, as defined in the appended claims. It has been found that this combination is particularly effective in alleviating or preventing the adverse effects of pollution on the skin. Thus the invention provides for the cosmetic use of compound (I) and anionic polymer (II) as an anti-pollutant agent for the skin, as defined in the appended claims.

The combination of compound (I) and anionic polymer (II) is described above in relation to compositions. The compositions described can be applied in the use provided for by the invention.

It has been found that the combination of compound (I) and anionic polymer (II) can help maintain the skin's morphology in the presence of pollutants. Thus, in one embodiment of the invention, there is provided use of the combination of compound (I) and anionic polymer (II) for the cosmetic treatment and/or care of the skin, wherein the cosmetic treatment/care is the maintenance and/or improvement of skin morphology, when the skin is exposed to pollutants.

In the context of this invention, "pollution" includes air and water pollution. Air pollution or pollutants can contain polycyclic aromatic hydrocarbons, volatile organic compounds, nitrogen oxides and particulate matter. Particulate matter contains heavy metals, minerals, organic toxins and/or biological materials. In particular, the invention is concerned with the effects of particulate matter on skin.

In another embodiment of the invention, the cosmetic treatment/care is the maintenance or improvement of skin smoothness, when the skin is exposed to pollutants.

In another embodiment of the invention, the cosmetic treatment/care is the maintenance or improvement of skin radiance and/or luminosity when the skin is exposed to pollutants.

In another embodiment of the invention, the cosmetic treatment/care is the maintenance or improvement of skin such that it appears less dull and looks more healthy when the skin is exposed to pollutants

In another embodiment of the invention, the cosmetic treatment/care is the alleviation or the prevention of the symptoms of premature skin aging due to exposure of the skin to pollutants, e.g. the treatment of skin wrinkles, the maintenance or smoothing out of skin wrinkles and/or the maintenance or prevention of pigment spots, when the skin is exposed to pollutants.

In another embodiment of the invention, the cosmetic treatment/care is the maintenance or improvement of skin cleanliness, when the skin is exposed to pollutants. A lack of cleanliness comes from the build up of impurities (e.g. city grime) in the skin due to pollution.

In another embodiment of the second aspect of the invention, the cosmetic treatment/care is the maintenance or improvement of skin oxygen levels, when the skin is exposed to pollutants.

In another embodiment of the second aspect of the invention, the cosmetic treatment/care is the maintenance or improvement of the skin's ability to breathe, when the skin is exposed to pollutants.

In another embodiment of the second aspect of the invention, the cosmetic treatment/care is the alleviation or prevention of adverse effects on the density of the skin's collagen network, when the skin is exposed to pollutants.

Also disclosed is a method of treatment and/or care of the skin which comprises the administration of the compositions described herein to the skin. The treatment and/or care is **cosmetic treatment and/or care as described herein.** In one embodiment, the administration is topical. **Application can be** carried out by iontophoresis, sonophoresis, electroporation, mechanical pressure, osmotic pressure gradient, occlusive cure, by microelectric patches, face masks or any combination thereof. The composition will comprise a cosmetically or pharmaceutically effective amount of compound (I) and anionic polymer (II).

The frequency of the application or administration of the combination of compound (I) and (II) can vary widely, depending on the needs of each subject, suggesting a range of application or administration from once per month to 10 times per day, from once per week to 4 times per day, from three times per week to three times per day, or once or twice per day.

Except in the Examples, or where otherwise explicitly indicated, all numerical quantities in this description specifying amounts of materials, reaction conditions, molecular weights, number of carbon atoms, and the like, are to be understood as approximated, i.e., subject to a variability of ± 5%, ± 3%, ± 1%, ± 0.1%, or ± 0.01% over the indicated value. It is to be understood that the upper and lower amount, range, and ratio limits set forth herein may be independently combined. Similarly, the ranges and amounts for each element of the technology described herein can be used together with ranges or amounts for any of the other elements.

The invention will now be illustrated by way of the following non-limiting examples.

### EXAMPLES

### Example 1: Preparation of a liposomal composition according to the invention

A liposomal composition is prepared, the ingredients of which are set out in table 1 below. In a suitable vessel, the ingredients of Phase A [INCI: WATER, PROPANEDIOL, GLYCERYL CAPRYLATE], are dissolved under rotor stirring and the mixture is heated at 30ºC until total solubilization of the glyceryl caprylate is achieved.

Subsequently, Phase A1 [INCI: XANTHAN GUM], is added until total dispersion.

In another beaker, the ingredients of Phase A2 [INCI: WATER, ACRYLIC ACID/ACRYLAMIDOMETHYL PROPANE SULFONIC ACID COPOLYMER], are dissolved under rotor stirring, this Phase A2 is added to the previous mixture.

Phase A3 [INCI: LECITHIN], is added to the previous mixture stirring under rotor until total solubilization is achieved.

In another beaker, the ingredients of Phase B [INCI: DIISOPROPYL ADIPATE, DIMETHYLMETHOXY CHROMANOL], are heated at 70 ºC until total solubilization of dimethylmethoxy chromanol is achieved. Phase B is added to the previous mixture stirring under rotor until total solubilization.

Phase C [INCI: SODIUM HYDROXIDE], is added to adjust the pH at 5.5 - 7.0.

**Table 1**

| INGREDIENT (INCI name) | % weight | Phase |
|---|---|---|
| WATER | 40.10 | A |
| PROPANEDIOL | 15.00 | A |
| GLYCERYL CAPRYLATE | 1.00 | A |
| XANTHAN GUM | 0.50 | A1 |
| WATER | 24.00 | A2 |
| [WATER, ACRYLIC ACID/ACRYLAMIDOMETHYL PROPANE SULFONIC ACID COPOLYMER (CG4500 POLYMER)] | 5.00 | A2 |
| LECITHIN | 4.00 | A3 |
| DIISOPROPYL ADIPATE | 9.00 | B |
| DIMETHYLMETHOXY CHROMANOL (LIPOCHROMAN^{®} *synthetic molecule)* | 1.00 | B |
| SODIUM HYDROXIDE | 0.40 | C |

### Example 2: Preparation of a cream composition containing the liposomal composition of the example 1

In a suitable vessel, the ingredients of Phase A [INCI: WATER, PROPANEDIOL, GLYCERYL CAPRYLATE], are dissolved under rotor stirring and the mixture is heated at 30ºC until a total solubilization of glyceryl caprylate is achieved.

Subsequently, Phase A1 [INCI: ACRYLATES/C10-30 ALKYL ACRYLATE CROSSPOLYMER], is added stirring under rotor until total dispersion.

Phase A2 [INCI: XANTHAN GUM], is then added stirring under rotor until total dispersion.

Phase A3 [INCI: POTASSIUM CETYL PHOSPHATE], is added stirring under rotor until total dispersion. This combined water phase [Phase A + Phase A1+ Phase A2 + Phase A3] is heated at 75-80ºC.

In another beaker, the ingredients of Phase B [INCI: METHYL GLUCOSE SESQUISTEARATE, STEARIC ACID, PEG-20 METHYL GLUCOSE SESQUISTEARATE, DIISOPROPYL ADIPATE], are heated at 75-80 ºC. Phase B is then added to the previous mixture [Phase A + Phase A1+ Phase A2 + Phase 3] stirring under turbine to make an emulsion.

Phase C, corresponding to the liposomal composition of Example 1 [INCI: [WATER, PROPANEDIOL, GLYCERYL CAPRYLATE, XANTHAN GUM, WATER, ACRYLIC ACID/ACRYLAMIDOMETHYL PROPANE SULFONIC ACID COPOLYMER, LECITHIN, DIISOPROPYL ADIPATE, DIMETHYLMETHOXY CHROMANOL, SODIUM HYDROXIDE], is added stirring under rotor until total dispersion when the mixture reaches 40ºC.

Phase D [INCI: SODIUM HYDROXIDE], is added to adjust the pH at 5.5 - 7.0.

The list of ingredients is in Table 2.

**Table 2**

| INGREDIENT (INCI name) | % weight | Phase |
|---|---|---|
| WATER | 70.10 | A |
| PROPANEDIOL | 10.00 | A |
| GLYCERYL CAPRYLATE | 1.00 | A |
| ACRYLATES/C10-30 ALKYL ACRYLATE CROSSPOLYMER | 0.30 | A1 |
| XANTHAN GUM | 0.30 | A2 |
| POTASSIUM CETYL PHOSPHATE | 1.00 | A3 |
| [METHYL GLUCOSE SESQUISTEARATE, STEARIC ACID] | 2.10 | B |
| PEG-20 METHYL GLUCOSE SESQUISTEARATE | 1.20 | B |
| DIISOPROPYL ADIPATE | 9.00 | B |
| LIPOSOMAL COMPOSITION OF THE EXAMPLE 1 | 5.00 | C |
| SODIUM HYDROXIDE | 0.40 | D |

### Study to evaluate the anti-pollution activity of the composition of the invention on human living skin explants after treatment by a pollutant mixture

The aim of this study is to evaluate the anti-pollution activity of the composition of the invention (referred to as the product) on human living skin explants after they have been treated with a pollutant mixture. After treatment of human skin explants ex vivo with the product and a solution containing heavy metals, hydrocarbons and diesel particles, anti-pollutant activity is evaluated by:
- Observation of the general morphology;
- Immunostaining of metallothionein-1 (MT-1H);
- Immunostaining of CD1a (Langerhans cells visualization); and
- Malondialdehyde (MDA) assay.

### Example 3: Treatment of human living skin explants with a pollutant mix.

An abdoplasty from a 54-year-old Caucasian woman is obtained and skin explants of an average diameter of 11 mm (±1 mm) are prepared. The explants are kept in survival in BEM culture medium (BIO-EC's Explants Medium) at 37°C in a humid, 5 %-CO₂ atmosphere.

For the explants being treated by the product, the product is topically applied on the basis of 2 µl per cm² (2 mg/explant), and spread using a small spatula, on days 0, 1, 2 and 3 (D0, D1, D2, and D3, respectively). The control explants (batch T0) do not receive any treatment except for renewal of the medium (1 ml of BEM medium per well).

Exposure to heavy metals, hydrocarbons and diesel particles is carried out topically on day 3 by treatment with a paper disk (9 mm ø) soaked with 30 µl of a solution containing a mixture of heavy metals, hydrocarbons and diesel particles for 24 hours. The mixture of pollutants is applied 4 hours after the application of the product. The product is the liposomal composition of Example 1 diluted at 5% in an assay vehicle (carboxy-methyl-cellulose aqueous gel).

On D0, 3 explants from batch T0 are collected and processed for histology and immunostaining.

On D4 (day 4), 3 explants from each batch are collected and processed for histology and immunostaining.

On D4, the culture medium of each explant is collected and stored at - 20°C for the MDA assay.

### Example 4: Evaluation of skin general morphology after treatment of human living skin explants with a pollutant mix.

After treatment of human skin explants ex vivo with the product and pollutants (heavy metals + diesel particles), activity is evaluated by observation of the histological general morphology (on D4).

After fixation for 24 hours in buffered formalin, the samples are dehydrated and impregnated in paraffin using a Leica TP 1010 dehydration automat. The samples are embedded using a Leica EG 1160 embedding station. 5-µm-thick sections are made using a Leica RM 2125 Minot -type microtome, and the sections are mounted on Superfrost^{®} histological glass slides.

Microscopical observations are made using a Leica DMLB or Olympus BX43 microscope. Pictures are digitized with a numeric DP72 Olympus camera with CellD storing software.

Observation of the general morphology is performed after staining of paraffinized sections according to Masson's trichrome, Goldner variant. The cellular and tissular morphology is evaluated on each cutaneous compartment (stratum corneum, living epidermis, dermal-epidermal junction, dermis). The observations are scored and the scores are shown in Table 3 below.

**Table 3**

| | Total score |
|---|---|
| Control without pollutants | 85 |
| Control + pollutants | 52 |
| Product + pollutants | 84 |

The results in Table 3 show that the product totally inhibits the alterations induced by a pollutant application on epidermal and dermal structure morphology. Thus, in presence of the product, the thickness of the stratum corneum, the epidermal and dermal-epidermal junction and the density of collagen network are protected against the alterations induced by pollutants and therefore the product is able to protect the skin from extrinsic aging ( and the symptoms thereof, e.g. wrinkles, loss of firmness and thickness) caused by pollution.

### Example 5: Metallothionein-1 immunostaining after treatment of human living skin explants with a pollutant mix.

Metallothioneins (MT) are proteins with high affinity to bind and to be induced by essential metals such as zinc and copper and harmful metals like cadmium and mercury. These proteins are believed to play an important role in the homeostatic function of essential metals and in detoxification against toxic metals.

After treatment of human skin explants ex vivo with the product and pollutants (heavy metals, hydrocarbons and diesel particles), anti-pollution activity is evaluated by metallothionein-1 (MT1-H) immunostaining (on D4).

After fixation for 24 hours in buffered formalin, the samples are dehydrated and impregnated in paraffin using a Leica TP 1010 dehydration automat. The samples are embedded using a Leica EG 1160 embedding station. 5-µm-thick sections are made using a Leica RM 2125 Minot -type microtome, and the sections are mounted on Superfrost^{®} histological glass slides.

MT1-H is stained on paraffinized section with a monoclonal anti-MT1-Hantibody (Dako, ref. M-0639, clone E9), diluted at 1:400 in PBS-BSA 0.3%-Tween 20% 0.05% for 1 hour at room temperature with an amplifyer system Vectastain RTU Universal VECTOR avidin/biotin and revealed with VIP (Vector Labs).

Microscopical observations are made using a Leica DMLB or Olympus BX43 microscope. Pictures are digitized with a numeric DP72 Olympus camera with CellD storing software.

The MT1-H staining is scored on a scale from 1 to 7 in order of increasing amount of MT1-H present, with 1 being the lowest amount. The scores are presented in Table 4 below.

**Table 4**

| | Total score |
|---|---|
| Control without pollutants | 3 |
| Control + pollutants | 5 |
| Product + pollutants | 2-3 |

The results show that the product is effective in inhibiting the MT-1H overexpression induced by pollutant application on the skin.

### Example 6: CD1a immunostaining after treatment of human living skin explants with a pollutant mix.

CD1a is a transmembrane glycoprotein, specifically expressed on Langerhans cell surface. It plays a key role as mediator of immune response. After contact with an allergenic compound, Langerhans cells migrate to the lymph nodes and they successively activate T cells. Thus a decrease in the amount of CD1a proteins in the skin can indicate that this migration has occurred.

After treatment of human skin explants ex vivo with the product and pollutants (heavy metals, hydrocarbons and diesel particles), anti-pollution activity is evaluated by CD1a immunostaining (on D4).

After fixation for 24 hours in buffered formalin, the samples are dehydrated and impregnated in paraffin using a Leica TP 1010 dehydration automat. The samples are embedded using a Leica EG 1160 embedding station. 5-µm-thick sections are made using a Leica RM 2125 Minot -type microtome, and the sections are mounted on Superfrost^{®} histological glass slides.

CD1a is stained on paraffinized section with a monoclonal anti-CD1a antibody (Novus Biological, ref: NBP2-34313, clone 010), diluted at 1:50 in PBS-BSA 0.3%-Tween 20% 0.05% for 1 hour at room temperature with an amplifyer system Vectastain RTU Universal VECTOR avidin/biotin and revealed with VIP (Vector Labs).

Microscopical observations are made using a Leica DMLB or Olympus BX43 microscope. Pictures are digitized with a numeric DP72 Olympus camera with CellD storing software.

Cd1a positive cells per cm of epidermis are observed in the epidermis and the results are presented in Table 5 below.

**Table 5**

| | Number of CD1a positive cells per cm of epidermis |
|---|---|
| Control without pollutants | 301 ± 2 |
| Control + pollutants | 214 ± 25 |
| Product + pollutants | 283 ± 23 |

The results show that the product induces a significant increase (32 %) of CD1a positive cells per cm of epidermis with respect to the control treated with pollutants.

### Example 7: Determination of MDA concentration in human living skin explants after treatment with a pollutant mix.

Lipid peroxidation is the degradation of lipids that occurs as a result of oxidative damage and is a useful marker for oxidative stress. Lipid peroxidation may contribute to the pathology of many diseases. Lipid peroxidation forms malondialdehyde (MDA) and 4-hydroxynonenal (4-HNE), as natural products. Measuring end products of lipid peroxidation is a useful measure of oxidative damage.

After treatment of human skin explants ex vivo with the product and pollutants (heavy metals, hydrocarbons and diesel particles), anti-oxidative damage activity is evaluated by MDA assay.

The MDA assay is carried out using an enhanced TBARS (Thiobarbituric acid reactive substances) assay method. The MDA is assayed in BEM medium by addition of TBARS solution (thiobarbituric acid, hydrochloric acid and tricholoroacetic acid). The mixture is placed in a water bath (80°c for 15 minutes). The MDA is extracted by a liquid/liquid extraction with butanol. The MDA in butanol is measured in spectrofluorimetry (excitation: 515 nm, emission: 550 nm) using a Tecan Infinite M200 Pro microplate reader.

Using this assay, MDA contained in the culture medium (BEM) on day 4 is measured for 4 explants per batch.

The MDA concentration is expressed in nmol/l in the results presented in Table 6 below.

**Table 6**

| | MDA (nmol/l) |
|---|---|
| Control without pollutants | 49.3 ± 2.6 |
| Control + pollutants | 125.3 ± 29.0 |
| Product + pollutants | 78.4 ± 12.9 |

The results show that the product induces a significant decrease (37 %) in MDA concentration with respect to the control treated with pollutants.

### Example 8: Test of the chelation capacity of the product by treatment with metal ion solution.

In recent years, pollution has become increasingly serious, so we are exposed to metals daily. They become dangerous to the body when they are metabolized or excreted and so accumulate in organs and tissues. One route of exposure is the absorption through the skin. The aim of this study is to investigate the chelating capacity of the product. The chelating capacity is investigated for each of the two active components of the composition of the invention and for the combination of the two active components when in contact to a solution of iron, nickel, zinc and lead.

Three different kinds of samples are prepared, in accordance with example 1, for this study:
(i) a liposomal composition containing 1.0 wt % LIPOCHROMAN^{®}, a synthetic molecule also referred to in this example as DIMETHYLMETHOXY CHROMANOL or Chromanol and the liposomal composition is also referred to in this example as "PMLs Chromanol" where PMLs stands for Plurilamellar Multivesicular Liposomes;
(ii) a liposomal composition containing 5.0 wt % polymer GC4500 also referred to in this example as [WATER, ACRYLIC AC-ID/ACRYLAMIDOMETHYL PROPANE SULFONIC ACID COPOLYMER] and the liposomal composition is also referred to in this example as "PMLs Polymer"; and
(iii) a liposomal composition containing 1.0 wt % LIPOCHROMAN^{®} and 5.0 wt % polymer GC4500, also refered to in this example as "PMLs Chromanol + Polymer".

A solution of metals is also prepared at 1ppm (parts per million) of each metal to be evaluated: iron (Fe), nickel (Ni), zinc (Zn) and lead (Pb). The selected concentration corresponds to the quantity of each metal in the environment according to the bibliography.

Samples are mixed with metal solution per triplicate at 5:95 proportions (5ml sample with 95ml metal solution). Different blanks are also made: a liposomal composition with only water (no metal solution) and metal solution with water (no liposomal composition). The mixtures are left for 6 hours, after that a dialysis is made in order to eliminate all the free metals (the ones that are not captured by the sample). A Float-A-Lyzer G2 (VWR) is used for every dialysis: a water solution is used to eliminate the free metals; this solution is renewed twice during the 20 hours of the experiment. The dialyzed samples are treated with HNO₃ 70% at 190ºC, and finally all of them are analyzed by ICP-MS (Inductively Coupled Plasma Mass Spectrometry).

The final concentrations in ppm obtained are shown in Table 7. These values represent the mean of 3 independent experiments.

**Table 7**

| **Sample** | **Fe** | **Ni** | **Zn** | **Pb** |
|---|---|---|---|---|
| **PMLs Polymer** | 0.83±0.02 | 0.90±0.01 | 0.82±0.11 | 0.83±0.02 |
| **PMLs Chromanol** | 0.78±0.01 | 0.91±0.01 | 0.77+0.02 | 0.86±0.01 |
| **PMLs Chromanol + Polymer** | 0.87±0.02 | 0.97±0.04 | 0.90±0.14 | 0.94±0.04 |

The final concentration indicates the value of the metals captured by the samples: a value close to 1 indicates a total capture of the metals and a value close to 0 indicates a null capture. Values >1 are within experimental error. The concentration of all the blanks was <0.1 (this value corresponds to the limit of quantification of the technique) and this value indicates the absence of metals in the original samples and the total elimination of free metals in case of no sample applied.

The results confirm the chelating capacity of DIMETHYLMETHOXY CHROMANOL (LIPOCHROMAN^{®} *synthetic molecule*) and ACRYLIC AC-ID/ACRYLAMIDOMETHYL PROPANE SULFONIC ACID COPOLYMER (in GC-4500 polymer) versus Fe, Ni, Zn and Pb.

### Example 9: Study of the protection from damage induced by Urban Dust on Normal Human Epidermal Keratinocytes from adult (NHEK) using a cell viability assay.

The capacity of protecting skin cell cultures from damage induced by Urban Dust (UD) is evaluated. The UD used is particulate matter (PMs, one of the major air pollutants with effects on the skin) collected in an urban area, Washington DC in 1976 and 1977, with an average particle size of 10.5 µm that contain polycyclic aromatic hydrocarbons (PAHs), polychlorinated biphenyl (PCBs), nitro-PAHs, chlorinated pesticides, dioxins and heavy metals. Damage is generated by the addition of UD to the culture medium and the protection effect from damage of the tested compounds is measured by a cell viability assay.

The viability is evaluated by MTT (3-[4,5-dimethylthiazol-2-yl]-2,5 diphenyl tetrazolium bromide), an absorbance-based cell viability method. Live cells present mitochondrial dehydrogenase activity that reduces the dye MTT to an insoluble blue colored product easily detected by absorbance, reflecting viable cells.

Normal Human Epidermal Keratinocytes from adult (NHEK) (PromoCell) are seeded at a density of 2×10⁴ cells/well in 96-clear well plates in culture medium. After 24 hours incubation at 37ºC in 5% CO₂ humidified air, the medium is removed and the cells are treated with fresh medium, DIMETHYLMETHOXY CHROMANOL (LIPOCHROMAN^{®} *synthetic molecule,* LPCH), [WATER, ACRYLIC ACID/ACRYLAMIDOMETHYL PROPANE SULFONIC ACID COPOLYMER] (CG4500 Polymer, POL) or a blend containing both of them (LPCH+POL), at different concentrations in culture medium during 24 hours. After incubation, UD (Standard Reference Material^{®} 1649b, National Institute of Standards and Technology, U.S. Department of Commerce) at 50 or 100 µg/mL is added and the cells are incubated for 24 hours to induce damage. Cells treated only with medium before incubation with UD are used as a control of damaged cells (control with pollutants). Cells treated with medium alone and not treated with UD are used as a control of living cells (control without pollutants). These cells (control without pollutants) are subjected to the same incubation regime as the other cells.

Then, cellular viability is evaluated. MTT (Sigma) is added to treated cells at a final concentration of 0.5 mg/mL per well and incubated for 3 hours at 37ºC in 5% CO₂ humidified air. After incubation, the plate is emptied, and 150 µl/well of DMSO (Sigma, dimethyl sulfoxide) are added to each well. Then, plates are shaken for 5 minutes at room temperature protected from light and absorbance read at 570 nm with wavelength correction at 630 nm, using a Microplate Absorbance Reader (Clariostar-BMG LabTech).

The percentage of cell viability for each condition is calculated respect to control without pollutants (cells treated with medium alone and not treated with UD). Test items are assayed in 4 independent experiments and each condition is assayed in 3-6 biological replicates.

**Table 8**

| **Treatment** | **Relative Cell Viability Respect To Control without pollutants (%)(MEAN ± SEM)** |
|---|---|
| Control without pollutants: Medium + Medium | 100.00 ± 1.67 |
| Control with pollutants: Medium + 50 µg/mL UD | 75.06 ± 3.11 |
| 50 µg/mL LPCH + 50 µg/mL UD | 85.81 ± 3.68 |
| 50 µg/mL POL + 50 µg/mL UD | 87.55 ± 3.22 |
| 50 µg/mL LPCH + 50 µg/mL POL+ 50 µg/mL UD | 103.50 ± 4.52 |
| 100 µg/mL POL + 50 µg/mL UD | 92.11 ± 3.03 |
| 50 µg/mL LPCH + 100 µg/mL POL+ 50 µg/mL UD | 95.08 ± 2.54 |
| 250 µg/mL POL + 50 µg/mL UD | 87.63 ± 2.56 |
| 50 µg/mL LPCH + 250 µg/mL POL+ 50 µg/mL UD | 91.71 ± 2.07 |
| 500 µg/mL POL + 50 µg/mL UD | 80.56 ± 2.51 |
| 50 µg/mL LPCH + 500 µg/mL POL+ 50 µg/mL UD | 90.22 ± 2.19 |

**Table 9**

| **Treatment** | **Relative Cell Viability Respect To Control without pollutants (%)(MEAN ± SEM)** |
|---|---|
| Control without pollutants: Medium + Medium | 100.00 ± 1.27 |
| Control with pollutants: Medium + 100 µg/mL UD | 62.57 ± 2.66 |
| 50 µg/mL LPCH + 100 µg/mL UD | 79.05 ± 3.21 |
| 50 µg/mL POL + 100 µg/mL UD | 87.93 ± 3.80 |
| 50 µg/mL LPCH + 50 µg/mL POL+ 100 µg/mL UD | 97.40 ± 3.80 |
| 100 µg/mL POL + 100 µg/mL UD | 85.31 ± 2.67 |
| 50 µg/mL LPCH + 100 µg/mL POL+ 100 µg/mL UD | 97.26 ± 4.19 |
| 250 µg/mL POL + 100 µg/mL UD | 85.80 ± 2.62 |
| 50 µg/mL LPCH + 250 µg/mL POL+ 100 µg/mL UD | 92.55 ± 3.26 |
| 500 µg/mL POL + 100 µg/mL UD | 82.65 ± 2.57 |
| 50 µg/mL LPCH + 500 µg/mL POL+ 100 µg/mL UD | 86.79 ± 1.93 |

The results show that either DIMETHYLMETHOXY CHROMANOL (LIPOCHROMAN^{®} *synthetic molecule*), ACRYLIC ACID/ACRYLAMIDOMETHYL PROPANE SULFONIC ACID COPOLYMER (in CG4500 Polymer) or the blend of both of them are able to increase the cell viability in NHEK cells culture respect to cells damaged by UD at the tested concentrations, showing a protection effect from damage induced by UD. In every case, the application of the blend of both substances induced a higher level of cell viability than the corresponding components applied separately.

### Example 10: In vivo study with the composition of Example 2, for the assessment of the barrier effect against environmental pollutants in Asian skin type female volunteers.

The short-term study is carried out during 1 day with measurements at initial time and after 6 hours of a single product application. 20 Asian female volunteers over 18 years old are included. Subjects apply the composition described in example 2 on one half-face (left or right) and a placebo cream on the other half-face. Both creams are only applied at initial time. The subjects serve as their own reference and results obtained at time 6 hours are compared with those obtained at initial time. Moreover, results obtained with the composition of example 2 are compared with those obtained with placebo cream.

The efficacy of the product is assessed by:
- Skin strips obtained in 2 different areas of each of the half-faces. Heavy metals (Iron (Fe), Lead (Pb), Chromium (Cr), Nickel (Ni) and Zinc (Zn)) contained in the skin stripping solution are quantified by means of graphite furnace atomic absorption spectroscopy (GF-AAS). Results are shown in table 10.

**Table 10. Percentage variations respect initial time of the concentration of heavy metals on strips after 6 hours of product application.**

| | **VARIATIONS (%) [(T+6 hours - T0)/T0]×100** | |
|---|---|---|
| | **Composition of Example 2** | **Placebo cream** |
| **Iron (Fe)** | **12.5%** | **77.4%** |
| **Lead (Pb)** | **-11.4%** | **73.8%** |
| **Chromium (Cr)** | **9.2%** | **41.2%** |
| **Nickel (Ni)** | **10.7%** | **72.5%** |
| **Zinc (Zn)** | **-4.3%** | **42.0%** |

These results shown in table 10 demonstrate that, after 6 hours of application of the composition of example 2, there is a significant decrease in the level of heavy metals accumulated on skin compared to placebo.

### Example 11: In vivo study with the composition of Example 2, for the assessment of the antioxidant efficacy in Asian skin type female volunteers.

The study is carried out during 30 days with measurements at initial time, and after 15 and 30 days of product application. 20 Asian female volunteers aged over 18 years old are included. Subjects apply the composition described in example 2 on one half-face (left or right) and a placebo cream on the other half-face. Both creams are applied twice a day (morning and night). The subjects serve as their own reference and results obtained at different times are compared with those obtained at initial time. Moreover, results obtained with the composition of example 2 are compared with those obtained with placebo cream.

The efficacy of the product is assessed by:
- Skin antioxidant capacity through measurement of ferric reducing antioxidant power (FRAP) on two strips obtained from each half-face of the volunteers' skin. Results are shown in table 11.

**Table 11. Percentage variations respect initial time of FRAP after 15 and 30 days of product application.**

| | **VARIATIONS (%)** | |
|---|---|---|
| | **[(T+15 days - T0)/T0]x100** | **[(T+30 days - T0)/T0]x100** |
| **FRAP - Composition of Example 2** | **12.6%** | **23.5%** |
| **FRAP - Placebo Cream** | **0.8%** | **1.4%** |

The results shown in table 11 demonstrate that, over a treatment period of 15 days, the composition described in example 2 is effective in preserving the skin antioxidant pool. Moreover, after 30 days of product application the skin antioxidant pool is even more preserved.
- Lipid peroxidation level through measurement of malonyldialdehyde (MDA) on one strip obtained from each half-face of the volunteers' skin. Results are shown in table 12.

**Table 12. Percentage variations respect initial time of the MDA concentration after 15 and 30 days of product application.**

| | **VARIATIONS (%)** | |
|---|---|---|
| | **[(T+15 days - T0)/T0]x100** | **[(T+30 days - T0)/T0]x100** |
| **MDA - Composition of Example 2** | **-8.2%** | **-24.7%** |
| **MDA - Placebo Cream** | **5.4%**** | **3.6%***** |

The results shown in table 12 demonstrate that, over a treatment period of 15 days the composition described in example 2 is effective in decreasing the level of lipid peroxides in the skin and, after 30 days of product application, the decrease is even higher.

### Example 12: In vivo study with the composition of Example 2, for the assessment of the protective effect against particles modelling atmospheric pollution.

The short-term study is carried out during 1 day. 21 Caucasian female volunteers between 21 and 45 years old are included. The composition described in example 2 is applied on a defined zone of the forearm while a second zone of the forearm is defined and remains not-treated. After product application, micro-particles modelling atmospheric pollution (particles sizes 1µm on average, Sun-PURO^{™} Black Iron Oxide, SunChemical) are applied on both defined zones. The quantity of micro-particles remaining on the skin after rinsing both areas is evaluated. Results obtained on the zone treated with the composition of example 2 are compared to those obtained on the non-treated zone.

The efficacy of the product is assessed by:
- Visualization with a videomicroscope being the surface (in pixels) covered by the particles (black areas) measured by image analysis. Results are shown in table 13.

**Table 13. Percentage in micro-particles quantity, after rinsing (t2) in comparison with the value measured before rinsing (t1) for each zone.**

| **Product** | **% of change (mean value)*** |
|---|---|
| Treated area | -50.2 |
| Non-treated area | -30.0 |

| | |
|---|---|
| ** Calculated as (t2-t1)*/*t1 × 100, where t2 corresponds to the quantity of micro-particles counted after rinsing and t1 corresponds to the same parameter before rinsing.* | |

From the results shown in table 13, the percentage of protection calculated as the absolute value of the subtraction of the % of change in the treated area minus the % of change in the non-treated area would be 20.2%. The results shown in table 13 demonstrate that, after application of the composition described in example 2, there is a significant decrease in adhesion of micro-particles modelling atmospheric pollution.

## Claims

1. Use of the combination of a compound (I) of formula (A) or (B): 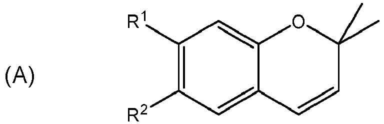 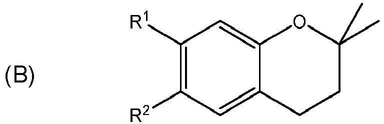 wherein R¹ and R² are identical or different and are independently selected from the group consisting of -OH, -CH₃, CF₃CH₂O- and CH₃O-; and an anionic polymer (II) which is a copolymer of acrylic acid or methacrylic acid and 2-acrylamido-2-methylpropane sulfonic acid, for the cosmetic treatment and/or care of the skin, wherein the cosmetic treatment and/or care is the alleviation or prevention of the adverse effects of air pollution containing particulate matter on the skin, said cosmetic treatment and/or care being the allevation and/or the prevention of the symptoms of premature aging due to exposure of the skin to pollutants, improvement of skin radiance and/or luminosity when the skin is exposed to pollutants, maintenance and/or improvement of skin morphology when the skin is exposed to pollutants, maintenance and/or improvement of the skin such that the skin appears less dull when the skin is exposed to pollutants, and/or maintenance and/or improvement of skin cleanliness when the skin is exposed to pollutants.

2. Use according to claims 1, wherein compound (I) is a compound of formula (B) in which R¹ is CH₃O- and R² is -OH.

3. Use according to any one of claims 1 to 2, wherein anionic polymer (II) is a copolymer of acrylic acid and 2-acrylamido-2-methylpropane sulfonic acid.

4. Use according to any one of claims 1 to 3, wherein the combination of the compound (i) of formula (A) or (B) and an anionic polymer (II) is in the form of a composition which contains 0.05 to 20 wt % of each of compound (I) and anionic polymer (II) with respect to the total weight of the composition.

5. Use according to any one of claims 1 to 4, wherein the compound (I) is present in an amount of from 0.01 to 0.10 wt % and the anionic polymer (II) is present in an amount from 0.05 to 0.5 wt% based on the total weight of the composition.

6. Use according to any one of claims 1 to 5, wherein the compound (I) and the anionic polymer (II) are present int the composition in a ratio of 1:5 by weight.

7. Use according to any one of claims 1 to 6, wherein the combination of the compound (i) of formula (A) or (B) and and anionic polymer (II) is in the form of a composition which contains at least one cosmetically or pharmaceutically acceptable excipient, adjuvant and/or ingredient.

8. Use according to claim 7, wherein compound (I) and anionic polymer (II) are incorporated into a cosmetically or pharmaceutically acceptable delivery system or sustained release system selected from the group formed by liposomes, mixed liposomes, oleosomes, niosomes, ethosomes, milliparticles, microparticles, nanoparticles and solid lipid nanoparticles, nanostructured lipid carriers, sponges, cyclodextrins, vesicles, micelles, mixed micelles of surfactants, surfactant-phospholipid mixed micelles, millispheres, microspheres and nanospheres, lipospheres, millicapsules, microcapsules, nanocapsules, microemulsions and nanoemulsions or is adsorbed on a solid organic polymer or solid mineral support selected from the group formed by talc, bentonite, silica, starch or maltodextrin.

9. Use according to claim 7 or claim 8, wherein this composition is presented in a formulation selected from the group formed by multiple emulsions, solutions, liquid crystals, anhydrous compositions, aqueous dispersions, oils, milks, balsams, foams, aqueous or oily lotions, aqueous or oily gels, creams, solutions, hydroalcoholic solutions, hydroglycolic solutions, hydrogels, liniments, sera, soaps, shampoos, conditioners, face masks, hairsprays, serums, polysaccharide films, ointments, mousses, pomades, pastes, powders, bars, pencils, sprays or aerosols.

10. Use according to any one of claims 1 to 9, wherein the combination of the compound (i) of formula (A) or (B) and and anionic polymer (II) is in the form of a composition and the composition is incorporated into a fabric, non-woven fabric or medical device.

11. Use according to any one of claims 7 to 10 wherein the combination of the compound (i) of formula (A) or (B) and and anionic polymer (II) is in the form of a composition which contains at least one cosmetically or pharmaceutically acceptable excipient, adjuvant and/or ingredient, wherein said excipient, adjuvant and/or ingredient is selected from the group formed by agents which diminish the sebum production, anti-seborrheic agents, mattifying agents, anti-acne agents, agents stimulating the synthesis of dermal or epidermal macromolecules and/or capable of inhibiting or preventing their degradation, collagen synthesis-stimulating agents, elastin synthesis-stimulation agents, decorin synthesis-stimulation agents, laminin synthesis-stimulation agents, defensin synthesis-stimulating agents, chaperone synthesis-stimulating agents, cAMP synthesis-stimulating agents, agents that modulate AQP-3, agents that modulate aquaporin synthesis, proteins from the aquaporin family, hyaluronic acid synthesis-stimulating agents, glycosaminoglycan synthesis-stimulating agents, fibronectin synthesis-stimulating agents, sirtuin synthesis-stimulating agents, heat shock proteins, heat shock protein synthesis-stimulating agents, agents which inhibit neuronal exocytosis, other anticholinergic agents, agents which inhibit muscular contraction, anti-aging agents, anti-wrinkle agents, antiperspirant agents, anti-inflammatory agents and/or analgesics, anti-itching agents, calming agents, anesthetic agents, inhibitors of acetylcholine-receptor aggregation, agents that inhibit acetylcholinesterase, skin relaxant agents, melanin synthesis stimulating or inhibiting agents, whitening or depigmenting agents, propigmenting agents, self-tanning agents, NO-synthase inhibiting agents, 5α-reductase inhibiting agents, lysyl- and/or prolyl hydroxylase inhibiting agents, antioxidants, free radical scavengers and/or agents against atmospheric pollution, reactive carbonyl species scavengers, anti-glycation agents, antihistamine agents, antiviral agents, antiparasitic agents, emulsifiers, emollients, organic solvents, liquid propellants, skin conditioners, humectants, substances that retain moisture, alpha hydroxy acids, beta hydroxy acids, moisturizers, epidermal hydrolytic enzymes, vitamins, amino acids, proteins, pigments or colorants, dyes, biopolymers, gelling polymers, thickeners, surfactants, softening agents, emulsifiers, binding agents, preservatives, agents able to reduce or treat the bags under the eyes, exfoliating agents, keratolytic agents, desquamating agents, antimicrobial agents, antifungal agents, fungistatic agents, bactericidal agents, bacteriostatic agents, agents stimulating the synthesis of lipids and components of the stratum corneum, ceramides, fatty acids, agents that inhibit collagen degradation, agents that inhibit matrix metalloproteinases, agents that inhibit elastin degradation, agents that inhibit serine proteases, agents stimulating fibroblast proliferation, agents stimulating keratinocyte proliferation, agents stimulating adipocyte proliferation, agents stimulating melanocyte proliferation, agents stimulating keratinocyte differentiation, agents stimulating or delaying adipocyte differentiation, antihyperkeratosis agents, comedolytic agents, anti-psoriasis agents, DNA repair agents, DNA protecting agents, stem cell protecting agents, stabilizers, agents for the treatment and/or care of sensitive skin, firming agents, anti-stretch mark agents, binding agents, lipolytic agents or agents stimulating lipolysis, adipogenic agents, agents modulating PGC-1α expression, agents modulating the activity of PPARγ, agents which increase or reduce the triglyceride content of adipocytes, anti-cellulite agents, agents which inhibit the activity of PAR-2, agents stimulating healing, coadjuvant healing agents, agents stimulating reepithelialization, coadjuvant reepithelialization agents, cytokine growth factors, agents acting on capillary circulation and/or microcirculation, agents stimulating angiogenesis, agents that inhibit vascular permeability, venotonic agents, agents acting on cell metabolism, agents which improve dermal-epidermal junction, agents inducing hair growth, hair growth inhibiting or retardant agents, hair loss retardant agents, preservatives, perfumes, odor absorbents and/or body odor masking deodorants, chelating agents, plant extracts, essential oils, marine extracts, agents obtained from a biotechnological process, mineral salts, cell extracts, sunscreens and organic or mineral photoprotective agents active against ultraviolet A and/or B rays and/or infrared A rays, or mixtures thereof.

12. Use according to any one of claims 1 to 11, wherein said cosmetic treatment and/or care of the skin comprises the topical administration to an individual of the combination of the compound (i) of formula (A) or (B) and and anionic polymer (II) or a composition comprising the combination of the compound (i) of formula (A) or (B) and and anionic polymer (II).

## Patentansprüche

1. Verwendung der Kombination aus einer Verbindung (I) der Formel (A) oder (B): 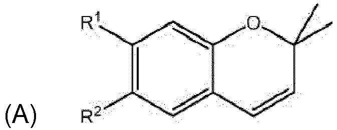 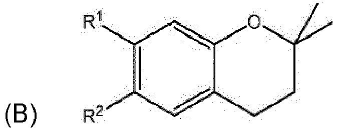 wobei R¹ und R² gleich oder verschieden sind und unabhängig voneinander aus der Gruppe bestehend aus -OH, -CH₃, CF₃CH₂O- und CH₃Oausgewählt sind; und ein anionisches Polymer (II), das ein Copolymer aus Acrylsäure oder Methacrylsäure und 2-Acrylamido-2-methylpropansulfonsäure ist, für die kosmetische Behandlung und/oder Pflege der Haut, wobei die kosmetische Behandlung und/oder Pflege die Linderung oder Vorbeugung der nachteiligen Wirkungen von partikelhaltiger Luftverschmutzung auf die Haut ist, wobei die kosmetische Behandlung und/oder Pflege die Linderung und/oder Vorbeugung der Symptome vorzeitiger Alterung aufgrund der Exposition der Haut gegenüber Schadstoffen ist, die Verbesserung der Ausstrahlung und/oder Leuchtkraft der Haut, wenn die Haut Schadstoffen ausgesetzt ist, die Aufrechterhaltung und/oder Verbesserung der Hautmorphologie, wenn die Haut Schadstoffen ausgesetzt ist, die Aufrechterhaltung und/oder Verbesserung der Haut, so dass die Haut weniger stumpf erscheint, wenn die Haut Schadstoffen ausgesetzt ist, und/oder die Aufrechterhaltung und/oder Verbesserung der Hautreinheit, wenn die Haut Schadstoffen ausgesetzt ist.

2. Verwendung nach Anspruch 1, wobei die Verbindung (I) eine Verbindung der Formel (B) ist, in der R¹ für CH₃O- und R² für -OH steht.

3. Verwendung nach einem der Ansprüche 1 bis 2, wobei das anionische Polymer (II) ein Copolymer aus Acrylsäure und 2-Acrylamido-2-methylpropansulfonsäure ist.

4. Verwendung nach einem der Ansprüche 1 bis 3, wobei die Kombination aus der Verbindung (i) der Formel (A) oder (B) und einem anionischen Polymer (II) in Form einer Zusammensetzung vorliegt, die 0,05 bis 20 Gew.-% von jeweils Verbindung (I) und anionischem Polymer (II), bezogen auf das Gesamtgewicht der Zusammensetzung, enthält.

5. Verwendung nach einem der Ansprüche 1 bis 4, wobei die Verbindung (I) in einer Menge von 0,01 bis 0,10 Gew.-% und das anionische Polymer (II) in einer Menge von 0,05 bis 0,5 Gew.-%, bezogen auf das Gesamtgewicht der Zusammensetzung, enthalten ist.

6. Verwendung nach einem der Ansprüche 1 bis 5, wobei die Verbindung (I) und das anionische Polymer (II) in der Zusammensetzung in einem Gewichtsverhältnis von 1:5 vorhanden sind.

7. Verwendung nach einem der Ansprüche 1 bis 6, wobei die Kombination aus der Verbindung (i) der Formel (A) oder (B) und dem anionischen Polymer (II) in Form einer Zusammensetzung vorliegt, die mindestens einen kosmetisch oder pharmazeutisch verträglichen Hilfsstoff, Adjuvans und/oder Inhaltsstoff enthält.

8. Verwendung nach Anspruch 7, wobei die Verbindung (I) und das anionische Polymer (II) in ein kosmetisch oder pharmazeutisch verträgliches Abgabesystem oder ein System mit verzögerter Freisetzung eingearbeitet sind, das ausgewählt ist aus der Gruppe, die gebildet wird von Liposomen, gemischten Liposomen, Oleosomen, Niosomen, Ethosomen, Millipartikeln, Mikropartikeln, Nanopartikeln und festen Lipid-Nanopartikeln, nanostrukturierten Lipidträgern, Schwämmen, Cyclodextrinen, Vesikeln Cyclodextrine, Vesikel, Mizellen, Tensid-Mischmizellen, Tensid-Phospholipid-Mischmizellen, Millikugeln, Mikrokugeln und Nanokugeln, Lipokugeln, Millikapseln, Mikrokapseln, Nanokapseln, Mikroemulsionen und Nanoemulsionen oder auf einem festen organischen Polymer oder einem festen Mineralträger aus der Gruppe Talk, Bentonit, Siliciumdioxid, Stärke oder Maltodextrin adsorbiert ist.

9. Verwendung nach Anspruch 7 oder 8, wobei diese Zusammensetzung in einer Formulierung vorliegt, die ausgewählt ist aus der Gruppe, die gebildet wird durch mehrfache Emulsionen, Lösungen, Flüssigkristalle, wasserfreie Zusammensetzungen, wässrige Dispersionen, Öle, Milche, Balsame, Schäume, wässrige oder ölige Lotionen, wässrige oder ölige Gele, Cremes, Lösungen, wässrig-alkoholische Lösungen, hydroglykolische Lösungen, Hydrogele, Einreibemittel, Seren, Seifen, Shampoos, Conditioner, Gesichtsmasken, Haarsprays, Seren, Polysaccharidfilme, Salben, Mousses, Pomaden, Pasten, Puder, Riegel, Stifte, Sprays oder Aerosole.

10. Verwendung nach einem der Ansprüche 1 bis 9, wobei die Kombination aus der Verbindung (i) der Formel (A) oder (B) und dem anionischen Polymer (II) in Form einer Zusammensetzung vorliegt und die Zusammensetzung in ein Gewebe, einen nicht gewebten Stoff oder eine medizinische Vorrichtung eingearbeitet ist.

11. Verwendung nach einem der Ansprüche 7 bis 10, wobei die Kombination der Verbindung (i) der Formel (A) oder (8) und des Anionpolymers (II) in Form einer Zusammensetzung vorliegt, die mindestens ein kosmetisch oder pharmazeutisch verträgliches Hilfsmittel und/oder einen kosmetisch oder pharmazeutisch verträglichen Inhaltsstoff enthält, wobei das Hilfsmittel, Adjuvans und/oder Inhaltsstoff ausgewählt ist aus der Gruppe, die gebildet wird durch Mittel, die zur Verringerung der Sebumproduktion beitragen, Antiseborrhoika, Mattierungsmittel, Wirkstoffe gegen Akne, Wirkstoffe, die zur Stimulation der Synthese der dermalen oder epidermalen Makromoleküle und/oder zur Hemmung oder Verhinderung ihres Abbaus geeignet sind, die Kollagensynthesestimulierende Wirkstoffe, Elastinsynthese-stimulierende Wirkstoffe, Decorinsynthese-stimulierende Wirkstoffe, Lamininsynthese-stimulierende Wirkstoffe, Defensinsynthese-stimulierende Wirkstoffe, Chaperonsynthesestimulierende Wirkstoffe, cAMP-Synthese-stimulierende Wirkstoffe, AQP-3-modulierende Wirkstoffe, die Aquaporin-Synthese modulierende Wirkstoffe, Proteine der Aquaporin-Familie, die Hyaluronsäure-Synthese stimulierende Wirkstoffe, die Glykosaminoglykan-Synthese stimulierende Wirkstoffe, die Fibronektin-Synthese stimulierende Wirkstoffe, die Sirtuin-Synthese stimulierende Wirkstoffe, Hitzeschock-Proteine, Hitzeschockproteine, welche die Synthese von Hitzeschockproteinen stimulieren, Wirkstoffe, die eine neuronale Exozytose hemmen, sonstige anticholinerge Wirkstoffe, Wirkstoffe, die eine Kontraktion der Muskulatur hemmen, Anti-Aging-Wirkstoffe, Mittel gegen Faltenbildung, schweißhemmende Wirkstoffe, entzündungshemmende und/oder schmerzstillende Wirkstoffe, Mittel gegen Juckreiz, Beruhigungsmittel, Anästhetika, Inhibitoren der Acetylcholinrezeptor-Aggregation, Wirkstoffe, die Acetylcholinesterase hemmen, hautentspannende Wirkstoffe, die Melaninsynthese stimulierende oder hemmende Wirkstoffe, aufhellende oder depigmentierende Wirkstoffe, propigmentierende Wirkstoffe, Selbstbräunungswirkstoffe, NO-Synthase-hemmende Wirkstoffe, 5α-Reduktase-hemmende Wirkstoffe, Lysyl- und/oder Prolylhydroxylase-hemmende Wirkstoffe, Antioxidantien, freie Radikalfänger und/oder Mittel gegen Luftverschmutzung, reaktive Carbonylspezies-Fänger, Antiglykierungsmittel, Antihistaminika, antivirale Wirkstoffe, Antiparasitika, Emulgatoren, Emollientien, organische Lösungsmittel, flüssige Treibmittel, Hautpflegemittel, Feuchthaltemittel, feuchtigkeitsbindende Stoffe, Alpha-Hydroxysäuren, Beta-Hydroxysäuren, Feuchthaltemittel, hydrolytische Enzyme der Epidermis, Vitamine, Aminosäuren, Proteine, Pigmente oder Farbmittel, Farbstoffe, Biopolymere, Gelpolymere, Verdickungsmittel, oberflächenaktive Stoffe, Weichmacher, Emulgatoren, Bindemittel, Konservierungsmittel, Wirkstoffe zur Verringerung oder Behandlung von Tränensäcken, Exfoliationsmittel, keratolytische Wirkstoffe, abschuppende Wirkstoffe, antimikrobielle Wirkstoffe, antimykotische Wirkstoffe, fungistatische Wirkstoffe, bakterizide Wirkstoffe, bakteriostatische Wirkstoffe, Wirkstoffe, die zur Synthese von Lipiden und Bestandteilen des Hornschichtgewebes anregen, Ceramide, Fettsäuren, Wirkstoffe, die den Kollagenabbau hemmen, Wirkstoffe, die Matrixmetalloproteinasen hemmen, Wirkstoffe, die den Elastinabbau hemmen, Wirkstoffe, die Serinproteasen hemmen, Wirkstoffe, die zur Fibroblastenproliferation anregen, Wirkstoffe, die zur Keratinozytenproliferation anregen, Wirkstoffe, die zur Adipozytenproliferation anregen, Wirkstoffe, welche die Melanozytenproliferation stimulieren, Wirkstoffe, die zur Differenzierung der Keratinozyten anregen, Wirkstoffe, die zur Differenzierung der Adipozyten anregen oder diese verzögern, Wirkstoffe gegen Hyperkeratose, Komedolytika, Wirkstoffe gegen Psoriasis, DNA-Reparaturmittel, DNA-Schutzmittel, Wirkstoffe zum Schutz der Stammzellen, Stabilisatoren, Wirkstoffe zur Behandlung und/oder Pflege empfindlicher Haut, Straffungsmittel, Mittel gegen Dehnungsstreifen, bindende Wirkstoffe, lipolytische Wirkstoffe oder Wirkstoffe, die eine Lipolyse stimulieren, adipogene Wirkstoffe, Wirkstoffe, die eine Modulation der PGC-1α-Expression bewirken, Wirkstoffe, die eine Modulation der Aktivität von PPARγ bewirken, Wirkstoffe, die den Triglyceridgehalt von Adipozyten erhöhen oder reduzieren, Anti-Cellulite-Wirkstoffe, Wirkstoffe, die eine Hemmung der Aktivität von PAR-2 bewirken, heilungsfördernde Wirkstoffe, koadjuvante heilungsfördernde Wirkstoffe, reepithelialisierungsfördernde Wirkstoffe, koadjuvante reepithelialisierungsfördernde Wirkstoffe, Zytokin-Wachstumsfaktoren, Wirkstoffe, die auf die Kapillarzirkulation und/oder Mikrozirkulation einwirken, Wirkstoffe, die zur Angiogenese anregen, Wirkstoffe, die eine Hemmung der Gefäßdurchlässigkeit bewirken, venotonische Wirkstoffe, Wirkstoffe, die auf den Zellstoffwechsel einwirken, Wirkstoffe, die den Übergang zwischen Dermis und Epidermis verbessern, Wirkstoffe, die das Haarwachstum fördern, Wirkstoffe, die das Haarwachstum hemmen oder verzögern, Wirkstoffe, die den Haarausfall verzögern, Konservierungsmittel, Parfümstoffe, Geruchsabsorber und/oder Deodorants, die den Körpergeruch überdecken, Chelatbildner, Pflanzenextrakte, ätherische Öle, Meeresextrakte, biotechnologisch gewonnene Wirkstoffe, Mineralsalze, Zellextrakte, Sonnenschutzmittel und organische oder mineralische lichtschützende Wirkstoffe, die gegen ultraviolette A- und/oder B-Strahlen und/oder Infrarot-A-Strahlen wirksam sind, oder deren Mischungen.

12. Verwendung nach einem der Ansprüche 1 bis 11, wobei die kosmetische Behandlung und/oder Pflege der Haut die topische Verabreichung der Kombination der Verbindung (i) der Formel (A) oder (B) und des anionischen Polymers (II) oder einer Zusammensetzung, welche die Kombination der Verbindung (i) der Formel (A) oder (B) und des anionischen Polymers (II) enthält, an eine Person umfasst.

## Revendications

1. Utilisation de la combinaison d'un composé (I) de formule (A) ou (B) : 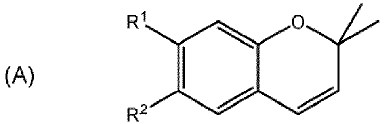 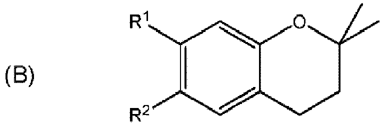 dans laquelle R¹ et R² sont identiques ou différents et sont indépendamment choisis dans le groupe constitué par -OH, -CH₃, CF₃CH₂O- et CH₃O- ; et d'un polymère anionique (II) qui est un copolymère d'acide acrylique ou d'acide méthacrylique et d'acide 2-acrylamido-2-méthylpropane sulfonique, pour les traitement et/ou soin cosmétiques de la peau, dans laquelle les traitement et/ou soin cosmétiques sont l'atténuation ou la prévention des effets indésirables de la pollution atmosphérique contenant des matières particulaires sur la peau, lesdits traitement et/ou soin cosmétiques étant l'atténuation et/ou la prévention des symptômes de vieillissement précoce du fait d'une exposition de la peau à des polluants, une amélioration de l'éclat et/ou de la luminosité de la peau lorsque la peau est exposée à des polluants, le maintien et/ou l'amélioration de la morphologie de la peau lorsque la peau est exposée à des polluants, le maintien et/ou l'amélioration de la peau de telle sorte que la peau apparaît moins terne lorsque la peau est exposée à des polluants, et/ou le maintien et/ou l'amélioration de la propreté de la peau lorsque la peau est exposée à des polluants.

2. Utilisation selon les revendications 1, dans laquelle le composé (I) est un composé de formule (B) dans laquelle R¹ est CH₃O- et R² est -OH.

3. Utilisation selon l'une quelconque des revendications 1 à 2, dans laquelle le polymère anionique (II) est un copolymère d'acide acrylique et d'acide 2-acrylamido-2-méthylpropane sulfonique.

4. Utilisation selon l'une quelconque des revendications 1 à 3, dans laquelle la combinaison du composé (I) de formule (A) ou (B) et d'un polymère anionique (II) est sous la forme d'une composition qui contient 0,05 à 20 % en poids de chacun du composé (I) et du polymère anionique (II) par rapport au poids total de la composition.

5. Utilisation selon l'une quelconque des revendications 1 à 4, dans laquelle le composé (I) est présent en une quantité allant de 0,01 à 0,10 % en poids et le polymère anionique (II) est présent en une quantité allant de 0,05 à 0,5 % en poids en fonction du poids total de la composition.

6. Utilisation selon l'une quelconque des revendications 1 à 5, dans laquelle le composé (I) et le polymère anionique (II) sont présents dans la composition dans un rapport de 1:5 en poids.

7. Utilisation selon l'une quelconque des revendications 1 à 6, dans laquelle la combinaison du composé (I) de formule (A) ou (B) et d'un polymère anionique (II) est sous la forme d'une composition qui contient au moins un excipient, adjuvant et/ou ingrédient acceptables sur le plan cosmétique ou pharmaceutique.

8. Utilisation selon la revendication 7, dans laquelle le composé (I) et le polymère anionique (II) sont incorporés dans un système d'administration ou système de libération prolongée acceptable sur le plan cosmétique ou pharmaceutique choisi dans le groupe formé par liposomes, liposomes mixtes, oléosomes, niosomes, éthosomes, milliparticules, microparticules, nanoparticules et nanoparticules lipidiques solides, supports lipidiques nanostructurés, éponges, cyclodextrines, vésicules, micelles, micelles mixtes d'agents tensioactifs, micelles mixtes d'agents tensioactifs et de phospholipides, millisphères, microsphères et nanosphères, liposphères, millicapsules, microcapsules, nanocapsules, microémulsions et nanoémulsions ou sont adsorbés sur un support polymère organique solide ou minéral solide choisi dans le groupe formé par talc, bentonite, silice, amidon ou maltodextrine.

9. Utilisation selon la revendication 7 ou la revendication 8, dans laquelle la présente composition est présentée dans une formulation choisie dans le groupe formé par émulsions multiples, solutions, cristaux liquides, compositions anhydres, dispersions aqueuses, huiles, laits, baumes, mousses, lotions aqueuses ou huileuses, gels aqueux ou huileux, crèmes, solutions, solutions hydro-alcooliques, solutions hydroglycoliques, hydrogels, liniments, sérums, savons, shampooings, après-shampooings, masques, laques capillaires, sérums, films de polysaccharides, onguents, mousses, pommades, pâtes, poudres, barres, crayons, sprays ou aérosols.

10. Utilisation selon l'une quelconque des revendications 1 à 9, dans laquelle la combinaison du composé (I) de formule (A) ou (B) et d'un polymère anionique (II) est sous la forme d'une composition et la composition est incorporée dans un tissu, une étoffe non tissée ou un dispositif médical.

11. Utilisation selon l'une quelconque des revendications 7 à 10 dans laquelle la combinaison du composé (I) de formule (A) ou (B) et d'un polymère anionique (II) est sous la forme d'une composition qui contient au moins un excipient, adjuvant et/ou ingrédient acceptables sur le plan cosmétique ou pharmaceutique, dans laquelle lesdits excipient, adjuvant et/ou ingrédient sont choisis dans le groupe formé par agents qui diminuent la production de sébum, agents anti-séborrhéiques, agents matifiants, agents anti-acnéiques, agents stimulant la synthèse de macromolécules dermiques ou épidermiques et/ou capables d'inhiber ou de prévenir leur dégradation, agents stimulant la synthèse de collagène, agents stimulant la synthèse d'élastine, agents stimulant la synthèse de décorine, agents stimulant la synthèse de laminine, agents stimulant la synthèse de défensine, agents stimulant la synthèse de chaperons, agents stimulant la synthèse d'AMPc, agents modulant la synthèse d'AQP-3, agents modulant la synthèse d'aquaporines, protéines de la famille des aquaporines, agents stimulant la synthèse d'acide hyaluronique, agents stimulant la synthèse de glycosaminoglycanes, agents stimulant la synthèse de fibronectine, agents stimulant la synthèse de sirtuines, protéines de choc thermique, agents stimulant la synthèse de protéines de choc thermique, agents inhibant l'exocytose neuronale, autres agents anticholinergiques, agents inhibant la contraction musculaire, agents anti-âge, agents anti-rides, agents anti-transpirants, agents anti-inflammatoires et/ou analgésiques, agents anti-démangeaisons, agents calmants, agents anesthésiques, inhibiteurs d'agrégation des récepteurs de l'acétylcholine, agents inhibiteurs de l'acétylcholinestérase, agents relaxants pour la peau, agents stimulant ou inhibant la synthèse de mélanine, agents blanchissants ou dépigmentants, agents propigmentants, agents autobronzants, agents inhibiteurs de la NO-synthase, agents inhibiteurs de la 5α-réductase, agents inhibiteurs de la lysyl- et/ou prolyl-hydroxylase, antioxydants, piégeurs de radicaux libres et/ou agents contre la pollution atmosphérique, piégeurs d'espèces carbonylées réactives, agents anti-glycation, agents antihistaminiques, agents antiviraux, agents antiparasitaires, émulsifiants, émollients, solvants organiques, agents propulseurs de liquides, revitalisants pour la peau, humectants, substances qui retiennent l'humidité, acides alpha-hydroxy, acides bêta-hydroxy, hydratants, enzymes hydrolytiques épidermiques, vitamines, acides aminés, protéines, pigments ou colorants, teintures, biopolymères, polymères gélifiants, épaississants, agents tensioactifs, agents adoucissants, émulsifiants, liants, conservateurs, agents capables de réduire ou de traiter les poches sous les yeux, agents exfoliants, agents kératolytiques, agents desquamants, agents antimicrobiens, agents antifongiques, agents fongistatiques, agents bactéricides, agents bactériostatiques, agents stimulant la synthèse de lipides et de composants de la couche cornée, céramides, acides gras, agents inhibiteurs de dégradation du collagène, agents inhibiteurs de métalloprotéinases matricielles, agents inhibiteurs de dégradation de l'élastine, agents inhibiteurs de sérines protéases, agents stimulant la prolifération de fibroblastes, agents stimulant la prolifération de kératinocytes, agents stimulant la prolifération d'adipocytes, agents stimulant la prolifération de mélanocytes, agents stimulant la différenciation de kératinocytes, agents stimulant ou retardant la différenciation d'adipocytes, agents antihyperkératose, agents comédolytiques, agents anti-psoriasis, agents réparateurs d'ADN, agents protecteurs d'ADN, agents protecteurs de cellules souches, stabilisateurs, agents pour le traitement et/ou le soin des peaux sensibles, agents raffermissants, agents anti-vergetures, agents liants, agents lipolytiques ou agents stimulant la lipolyse, agents adipogéniques, agents modulant l'expression de PGC-1α, agents modulant l'activité de PPARγ, agents augmentant ou réduisant la teneur en triglycérides des adipocytes, agents anti-cellulite, agents inhibant l'activité de PAR-2, agents stimulant la cicatrisation, agents coadjuvants de cicatrisation, agents stimulant la réépithélialisation, agents coadjuvants de réépithélialisation, facteurs de croissance cytokiniques, agents agissant sur la circulation capillaire et/ou la microcirculation, agents stimulant l'angiogenèse, agents inhibant la perméabilité vasculaire, agents veinotoniques, agents agissant sur le métabolisme cellulaire, agents améliorant la jonction dermo-épidermique, agents induisant la croissance des cheveux, agents inhibiteurs ou retardateurs de croissance des cheveux, agents retardateurs de chute des cheveux, conservateurs, parfums, absorbants d'odeurs et/ou déodorants masquant les odeurs corporelles, agents chélatants, extraits végétaux, huiles essentielles, extraits marins, agents issus d'un processus biotechnologique, sels minéraux, extraits de cellules, écrans solaires et agents photoprotecteurs organiques ou minéraux actifs contre les rayons ultraviolets A et/ou B et/ou les rayons infrarouges A, ou mélanges de ceux-ci.

12. Utilisation selon l'une quelconque des revendications 1 à 11, dans laquelle lesdits traitement et/ou soin cosmétiques de la peau comprennent l'administration topique à un sujet de la combinaison du composé (I) de formule (A) ou (B) et d'un polymère anionique (II) ou d'une composition comprenant la combinaison du composé (I) de formule (A) ou (B) et d'un polymère anionique (II).
